(19) Europäisches Patentamt
European Patent Office
Office européen des brevets

(11) Veröffentlichungsnummer : **0 064 629**
**B1**

(12) # EUROPÄISCHE PATENTSCHRIFT

(45) Veröffentlichungstag der Patentschrift : **26.10.83**

(51) Int. Cl.³ : **C 07 C 97/24**, C 07 C101/80, C 07 C 85/11

(21) Anmeldenummer : **82103362.8**

(22) Anmeldetag : **21.04.82**

(54) **Verfahren zur Herstellung von Aminoanthrachinonen.**

(30) Priorität : **07.05.81 DE 3118020**

(43) Veröffentlichungstag der Anmeldung : **17.11.82 Patentblatt 82/46**

(45) Bekanntmachung des Hinweises auf die Patenterteilung : **26.10.83 Patentblatt 83/43**

(84) Benannte Vertragsstaaten : **BE CH DE FR GB LI**

(56) Entgegenhaltungen :
**DE A 2 164 458**
**DE B 1 543 605**
**DE B 2 557 442**

**CHEMICAL & PHARMACEUTICAL BULLETIN, Band 28, August 1980 MASANORI SOMEI et al. « Titanium- (III) Chloride for the Reduction of Heteroaromatic and Aromatic Nitro Compounds » Seiten 2515-2518**

(73) Patentinhaber : **BASF Aktiengesellschaft**
**Carl-Bosch-Strasse 38**
**D-6700 Ludwigshafen (DE)**

(72) Erfinder : **Puetter, Hermann, Dr. Chem.**
**Landauer Strasse 59**
**D-6730 Neustadt (DE)**

Anmerkung : Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Jouve, 18, rue St-Denis, 75001 Paris, France

EP 0 064 629 B1

# Verfahren zur Herstellung von Aminoanthrachinonen

Die Erfindung betrifft ein Verfahren zur Herstellung von Aminoanthrachinonen durch Reduktion von Nitroanthrachinonen mit Titan-(III)-salzen bei einem pH unterhalb 2 und Abtrennung des Endstoffs bei einem pH von unterhalb 2.

Aminoanthrachinone werden in der Regel aus den entsprechenden Nitroanthrachinonen gewonnen. Die im allgemeinen anwendbare Methode ist die der Reduktion. Hierfür können die unterschiedlichsten Reduktionsmittel verwendet werden (Houben-Weyl, Methoden der Organischen Chemie, 7/3 c (1979), Seiten 158 bis 161). Es sind auch Verfahren bekannt, Nitroanthrachinone katalytisch zu hydrieren. Aufgrund der Schwerlöslichkeit von Nitroanthrachinonen und der Empfindlichkeit vieler Nitroanthrachinonderivate gelingt die Reduktion nicht immer glatt oder es treten Komplikationen infolge von Nebenprodukten auf. Das am meisten verwendete Reduktionsmittel ist Natriumsulfid ($Na_2S$ oder $NaHS$). Neben den Problemen durch Nebenreaktionen fordert hier vor allem die Toxizität von $H_2S$ besondere Maßnahmen bezüglich der Arbeitssicherheit. Außerdem belastet die Mutterlauge das Abwasser. Andere Reduktionsmittel wie $SnCl_2$ oder $NaBH_4$ scheiden aus Kostengründen aus. Hydrazin ist sowohl teuer als auch giftig. Die katalytische Hydrierung bereitet wegen der Schwerlöslichkeit der Verbindungen sowie der schwierigen Katalysator-Abtrennung technische Probleme.

Die reduzierende Wirkung von Ti(III)-salzen auf Nitrochlorbenzaldehyd (Ausbeute 53,4 %) ist bekannt (Ber. dtsch. chem. Ges. *37*, 1873 (1904)). Weiterhin lassen sich Nitrobenzole (Synthesis *1974*, 45 ; Chem. Pharm. Bull. *28* (1980), 2 515-2 518) in die entsprechenden Amine überführen. Aufgrund der irreversiblen Hydrolyse, die die Titansalze bei der Aufarbeitung der Reaktionsansätze erleiden, ist diese Methode aber allenfalls für analytische Zwecke oder im Labormaßstab geeignet. Zur Isolierung müssen die Endprodukte — die z. T. zunächst als Komplexe von Titansalzen anfallen — nämlich zuerst durch Anheben des pH freigesetzt werden ; bei dieser Überführung der Aminsalze in die freien Basen entsteht ein Hydroxidniederschlag. Pro Mol Wertprodukt muß mit einem Zwangsanfall von 6 Mol verunreinigtem Titanoxidhydrat gerechnet werden.

Es wurde nun gefunden, daß man Aminoanthrachinone durch Reduktion vorteilhaft erhält, wenn man Nitroanthrachinone mit Titan-(III)-salzen bei einem pH unterhalb 2 reduziert und den Endstoff bei einem pH von unterhalb 2 abtrennt.

Die Umsetzung kann für den Fall der Verwendung von Titan-III-chlorid, Salzsäure und 1-Nitroanthrachinon durch die folgenden Formeln wiedergegeben werden :

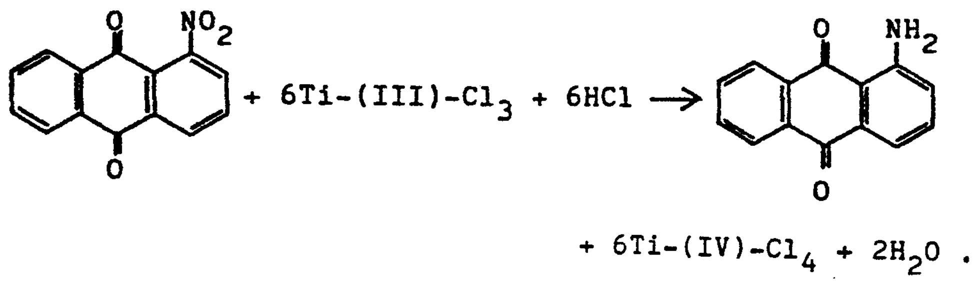


Im Vergleich mit dem Stand der Technik liefert das erfindungsgemäße Verfahren auf einfacherem und wirtschaftlicherem Wege eine große Zahl von Aminoanthrachinonen in besserer Ausbeute und Reinheit. Überraschend lassen sich die entstehenden Aminoanthrachinone nicht nur leicht durch einfache Filtration abtrennen, sondern auch leicht aus den zunächst entstehenden Titankomplexen freisetzen. Das erfindungsgemäße Verfahren reduziert Nitroanthrachinone ohne Komplikationen durch Nebenreaktionen und unter Vermeidung einer Umweltbelastung, besonders der von Titanoxidschlamm.

Die entstehende Titan-(IV)-salz-Mutterlauge kann regeneriert und dem Reduktionsprozeß wieder zugeführt werden. Die Regenerierung einer solchen Titansalzlösung oder die Herstellung einer Titan-(III)-Lösung erfolgt besonders vorteilhaft durch kathodische Reduktion. Alle diese vorteilhaften Ergebnisse sind im Hinblick auf die bekannten Verfahren überraschend.

Bevorzugte Aminoanthrachinone sind solche der Formel

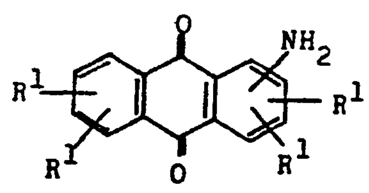
(I)

worin die einzelnen Reste $R^1$ gleich oder verschieden sein können und jeweils ein wasserstoffatom, ein Halogenatom, insbesondere ein Bromatom und vorzugsweise ein Chloratum, einen aliphatischen Rest,

insbesondere einen Alkylrest mit 1 bis 6 Kohlenstoffatomen, eine Hydroxygruppe, eine Carboxylgruppe, eine Alkoxygruppe, vorzugsweise mit 1 bis 6 Kohlenstoffatomen, eine Sulfonsäuregruppe bedeuten, darüber hinaus ein, zwei oder drei Reste $R^1$ auch jeweils für eine weitere Aminogruppe stehen können. Dementsprechend sind bevorzugte Nitroanthrachinone solche der Formel

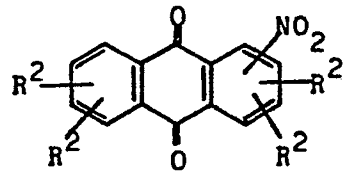

(II)

worin die einzelnen Reste $R^2$ die Bedeutung von $R^1$ besitzen oder, sofern Reste $R^1$ jeweils eine Aminogruppe bezeichnen, die entsprechenden Reste $R^2$ auch jeweils für eine Nitrogruppe stehen können. Die vorgenannten Reste können noch durch unter den Reaktionsbedingungen inerte Gruppen, z. B. Aminogruppen, Alkyl- oder Alkoxygruppen mit jeweils 1 bis 4 Kohlenstoffatomen, substituiert sein.

So kommen beispielsweise als Ausgangsstoffe II in Frage : 1-Nitroanthrachinon, 2-Nitroanthrachinon ; in 4-Stellung durch Brom, Chlor, eine Methyl-, Ethyl-, Propyl-, Isopropyl-, Butyl-, Isobutyl-, sek.-Butyl-, tert.-Butyl-gruppe, Hydroxygruppe, Carboxygruppe, Methoxy-, Ethoxy-, Propoxy-, Isopropoxy-, Butoxy-, Isobutoxy-, sek.-Butoxy-, tert.-Butoxy-gruppe substituiertes 1- oder 2-Nitroanthrachinon ; entsprechend in 1-, 2-, 3-, 5-, 6-, 7-, 8-Stellung substituierte 1- bzw. 2-Nitroanthrachinone ; durch entsprechende Substituenten in 1,3-, 1,4-, 2,3-, 2,4-, 1,5-, 2,6-, 1,8-, 4,5-Stellung substituierte 1- oder 2-Nitroanthrachinone ; entsprechende in vorgenannten Stellungen 3- oder 4-fach durch vorgenannte Substituenten substituierte 1- oder 2-Nitroanthrachinone ; entsprechende unsubstituierte oder substituierte 1,2-, 1,4-, 2,3-, 1,5-, 2,6-, 1,8-, 4,5-Dinitroanthrachinone und 1,4,5- und 1,4,8-Trinitroanthrachinone. Bevorzugt sind : 1-Nitroanthrachinon, 2-Nitroanthrachinon, 1-Nitro-2-methylanthrachinon, 1,4-Dinitroanthrachinon-2-carbonsäure, 1-Nitroanthrachinon-2-Carbonsäure, 1-Nitro-4-amino-anthrachinon-2-carbonsäure, 1-Nitro-5-chloranthrachinon.

Das Titansalz kann mit dem Nitroanthrachinon in stöchiometrischer Menge oder im Überschuß, vorzugsweise in einem Verhältnis von 6,1 bis 10, insbesondere von 6,3 bis 7 Mol Titan-(III)-salz je Mol Nitrogruppe im Nitroanthrachinon umgesetzt werden. Gegebenenfalls kann man bei Di- und Polynitroverbindungen durch entsprechend stöchiometrisch unterschüssige Mengen an Titan-(III)-salz Nitroaminoverbindungen erhalten. Als Titan-(III)-salze kommen z. B. in Frage : Titan-(III)-fluoroborat, -bromid, -jodid, -fluorid, -sulfat, -formiat und bevorzugt -chlorid.

Als Reaktionsmedium kommen wäßrige, saure Lösungen von Titan-(III)-salzen in Frage. Die Konzentration der Titansalze liegt zweckmäßig zwischen 0,05 und 3 molar, vorzugsweise 0,5 bis 2 molar, insbesondere 1,4 bis 1,8 molar. Die Umsetzung wird in Gegenwart von Säure, vorteilhaft einer Menge von 1 bis 5, insbesondere von 1,5 bis 3,5 Äquivalenten Säure, bezogen auf 1 Mol Ausgangsstoff II, durchgeführt. Es werden in der Regel anorganische Säuren verwendet. Anstelle einbasischer Säuren können auch äquivalente Mengen mehrbasischer Säuren zur Anwendung gelangen. Beispielsweise sind folgende Säuren geeignet : Chlorwasserstoff, Bromwasserstoff, Jodwasserstoff, Perchlorsäure, Schwefelsäure, Phosphorsäure ; Sulfonsäuren wie Benzol- und p-Toluolsulfonsäure ; Bor enthaltende Säuren wie Borfluorwasserstoffsäure ; die bevorzugten Säuren sind $H_2SO_4$, HCl, HBr und $HBF_4$. Ein besonders günstiges Medium ist die Lösung von $TiCl_3$ in wäßriger HCl. Zweckmäßig sind 10 bis 25, insbesondere 10 bis 15 Gewichtsprozent wäßrige Lösungen der Säure als Reaktionsmedium. Die Lösung kann vor oder während der Reaktion mit $H_2O$ oder wäßriger Säure, z. B. HCl, verdünnt werden.

Die Umsetzung wird zweckmäßig bei einer Reaktionstemperatur von + 10 bis + 120 °C, vorzugsweise von + 25 bis + 80 °C, drucklos oder unter Druck, kontinuierlich oder diskontinuierlich durchgeführt. Zuweilen ist es vorteilhaft, bei der erfindungsgemäßen Umsetzung einen Emulgator zuzusetzen. Es kommen dabei neutrale, anionische oder kationische Tenside in Betracht. Die Menge des zugesetzten Tensids liegt zwischen 0,1-10 Gewichtsprozent, vorzugsweise 0,5-2 Gewichtsprozent, bezogen auf den Ausgangsstoff II.

Geeignete Tenside sind beispielsweise Ethoxylate von $C_9$-$C_{15}$-Oxoalkoholen, Fettalkoholethoxylate, Alkylphenolethoxylate, Dodecylbenzolsulfonsäure, Schwefelsäurehalbester von $C_9$-$C_{15}$-Alkoholen, Alkylnaphthalinsulfonsäuren, Tetraalkylammoniumsalze oder Trialkylamine, die mindestens einen langkettigen Alkylrest enthalten.

Die Reaktion kann wie folgt durchgeführt werden : Ein Gemisch von Ausgangsstoff II, Titan-(III)-salz in wäßrigem Säuremedium, gegebenenfalls in Gegenwart eines Emulgators, wird während 0,25 und 15 Stunden, vorzugsweise zwischen 0,5 und 6 Stunden, bei der Reaktionstemperatur gehalten. Es ist manchmal vorteilhaft, zunächst bei niedriger Temperatur zu beginnen, z. B. bei 30 °C, und später auf höhere Temperaturen aufzuheizen. Der Verlauf der Reaktion kann durch titrimetrische oder polarographische Bestimmung des Titan-(III)-salzes bequem verfolgt werden. Da das Reagenz sehr selektiv arbeitet, ist nur ein geringer Überschuß von 5 Gew.% bis 30 Gew.% bezogen auf die theoretische Menge für einen vollständigen Umsatz nötig. Das erfindungsgemäß bevorzugte System $TiCl_3$ in wäßriger HCl ist im bevorzugten konzentrationsbereich fast völlig stabil gegen Luftsauerstoff (Beispiel 6).

Die Aufarbeitung gestaltet sich bei den vorliegenden Verbindungen mit Ti-(III)-salzen im Vergleich zur Reduktion von anderen Nitroaromaten als besonders einfach. Der Endstoff wird durch Filtration, Absaugen oder Abschleudern in üblicher Weise gewonnen. Die Mutterlauge kann direkt zur Regenerierung von $Ti^{3+}$ eingesetzt werden. Die je nach Beschaffenheit des Filterrückstandes mehr oder weniger großen Waschlösungsmengen werden entweder verworfen bzw. ganz oder teilweise ebenfalls der Regenerierung zugeführt. Während der Reaktion wird ein pH unterhalb 2, vorteilhaft unterhalb 1, eingehalten, bei der Abtrennung des Endstoffs vom Reaktionsgemisch bzw. Aufarbeitungsgemisch durch z. B. beim Absaugen oder Verdünnen mit Eiswasser, das pH auf unterhalb 2, vorzugsweise unterhalb 1, eingehalten.

Die nach dem Verfahren der Erfindung erhältlichen Aminoanthrachinone sind wertvolle Ausgangsstoffe für Schädlingsbekämpfungsmittel, Pharmazeutika und insbesondere Farbstoffe. Bezüglich der Verwendung wird auf die vorgenannte Literatur und Ullmanns Encyklopädie der technischen Chemie, 4. Ausgabe, Band 7, Seiten 590 bis 600, verwiesen.

Die in den folgenden Beispielen aufgeführten Teile bedeuten Gewischtsteile. Die Gewichtsteile verhalten sich zu den Volumenteilen wie Kilogramm zu Liter.

Beispiel 1

Herstellung von 1-Aminoanthrachinon

a) Herstellung einer Titantrichloridlösung durch kathodische Reduktion

Für die Herstellung von $TiCl_3$ wird eine geteilte Platten- und Rahmen-Zelle verwendet. Die Zelle besteht aus zwei Graphitelektroden (aktive Fläche je 1 $dm^2$), zwischen denen sich zwei rechteckige PVC-Rahmen und eine Kationenaustauschermembran befindet. Die Membran, ein Rahmen und je eine Elektrode bilden den Kathoden- bzw. den Anodenraum der Zelle. Die Rahmen sind jeweils mit zwei Stutzen für Zu- und Ablauf des entsprechenden Elektrolyten ausgerüstet. Anolyt und Katholyt werden mit zwei Magnetkreiselpumpen über einen Glaskühler und einen Vorratsbehälter durch die Zelle im Kreis gepumpt. Bei einer Stromstärke von 5 A wird eine Lösung aus 250 Teilen HCl konz., 750 Teilen $H_2O$ und 700 Teilen $TiCl_4$ als Katholyt (Anolyt : wäßrige HCl) bei etwa 30 °C durch die Zelle gepumpt. Nach etwa 15 Stunden sind bereits 75 % der $TiCl_4$-90 %. Nach 24 Stunden wird abgebrochen, $TiCl_4$ hat sich nahezu vollständig zu $TiCl_3$ umgesetzt.

Anschließend wird die Lösung aus dem Katholytkreislauf abgelassen, der Kreislauf wird mit wenig verdünnter HCl gespült und die Spüllösung mit der Katholytlösung vereinigt.

In ähnlicher Weise wird auch eine ausreagierte $TiCl_4$-Mutterlauge, evtl. nach teilweiser Abdestillation von $H_2O$ regeneriert.

b) 13,3 Teile 1-Nitroanthrachinon werden in 100 Teilen $H_2O$, 100 Teilen HCl konz. (36 Gew.%) vorgelegt und unter Rühren mit 190 Teilen $TiCl_3$-Lösung, die entsprechend Beispiel 1a) hergestellt wird und 54 Teile $TiCl_3$ enthält, versetzt. Nach 3 Stunden Rühren bei Raumtemperatur wird auf 60 °C aufgeheizt, nochmal 3 Stunden gerührt, anschließend auf Eis gegeben, der Endstoff abgesaugt, gewaschen und getrocknet. Das pH der Reaktion ist < 1, das pH der Abtrennung des Endstoffs ist ebenfalls < 1.

Ausbeute : 9,5 Teile (81 % der Theorie) 1-Aminoanthrachinon, Fp 248-250 °C.

Beispiel 2

Herstellung von 1-Amino-2-methylanthrachinon

13,3 Teile 1-Nitro-2-methylanthrachinon werden mit 378 Teilen wäßriger $TiCl_3$-Lösung, enthaltend 54 Teile $TiCl_3$, bei 65 °C 3 Stunden gerührt. Der Verlauf der Reaktion wird durch Titration von $TiCl_3$ mit 0,1-m-$FeCl_3$-Lösung verfolgt. Nachdem $TiCl_3$ verbraucht ist, wird abgekühlt, der Endstoff abgesaugt und getrocknet. Das pH der Reaktion ist < 1, das pH der Abtrennung des Endstoffs ist 1.

Ausbeute : 9,34 Teile (79 % der Theorie) 1-Amino-2-methylanthrachinon, Fp 195 °C.

Beispiel 3

Herstellung von 1,4-Diaminoanthrachinoncarbonsäure-2

7,80 Teile 1,4-Dinitroanthrachinon-2-carbonsäure, 0,1 Teile oxäthyliertes Nonylphenol mit 8 Äthoxygruppen, 50 Teile $H_2O$, 50 Volumenteile HCl konz. (36 Gew.%) werden mit 110 Teilen einer Titan-(III)-chlorid-Lösung (Gehalt 1,69 Mol/kg) versetzt und 8 Stunden bei Raumtemperatur gerührt. Der entstehende Niederschlag wird abgesaugt, neutralgewaschen und in 20-gewichtsprozentiger NaOH aufgenommen. Nach Verdünnen mit $H_2O$ wird abgesaugt, mit Eisessig angesäuert; der entstehende Festkörper wird abgesaugt und getrocknet. Das pH der Reaktion ist < 1, das pH der Abtrennung des Endstoffs ist < 2.

Ausbeute : 4,7 Teile (67 % der Theorie) 1,4-Diaminoanthrachinoncarbonsäure-2, Fp < 350 °C.

Beispiel 4

Herstellung von 1-Aminoanthrachinoncarbonsäure-2

12 Teile 1-Nitroanthrachinoncarbonsäure-2 werden mit 223 Teilen einer Titan-(III)-chlorid-Lösung in HCl (21 Teile $TiCl_3$) und 0,1 Teilen oxäthyliertes Nonylphenol mit 8 Äthoxygruppen auf 60 °C aufgeheizt und 4,5 Stunden bei dieser Temperatur gerührt. Der entstehende Festkörper wird abgesaugt, mit $H_2O$ gewaschen, mit verdünnter HCl ausgerührt. Dann wird erneut abgesaugt, mit $H_2O$ neutralgewaschen und getrocknet. Das pH der Reaktion ist < 1, das pH der Abtrennung des Endstoffs ist 1,9.

Ausbeute : 8,7 Teile (82 % der Theorie) 1-Aminoanthrachinoncarbonsäure-2, Fp 285 °C.

Beispiel 5

Herstellung von 1-Amino-5-chloranthrachinon

12 Teile 1-Chlor-5-nitroanthrachinon und 0,1 Teile oxäthyliertes Nonylphenol mit 8 Äthoxygruppen werden mit 223 Teilen einer Titan-(III)-chlorid-Lösung in HCl (21 Teilen $TiCl_3$) erst eine Stunde bei Raumtemperatur, dann 3,5 Stunden bei 60 °C kräftig gerührt. Der entstehende Festkörper wird abgesaugt, mit heißer, wäßriger HCl ausgerührt, erneut abgesaugt, neutralgewaschen und getrocknet. Das pH der Reaktion ist < 1, das pH der Abtrennung des Endstoffs ist < 1.

Rückstand : 10,2 Teile (95 % der Theorie) 1-Amino-5-chloranthrachinon, Fp 205-209 °C.

Beispiel 6

Stabilität einer $TiCl_3$-Lösung

In 250 Volumenteilen einer nach Beispiel 1a) hergestellten $TiCl_3$-Lösung werden unter Rühren bei 50 °C über einen Zeitraum von 20 Stunden 1 180 Volumenteile feuchte Luft eingeleitet. Die Lösung enthält zu Beginn 65 Teile $TiCl_3$/Volumenteil Lösung, am Ende 62 Teile $TiCl_3$/Volumenteil Lösung, das Volumen der Lösung hay sich nicht geändert.

**Anspruch**

Verfahren zur Herstellung von Aminoanthrachinonen durch Reduktion, dadurch gekennzeichnet, daß man Nitroanthrachinone mit Titan-(III)-salzen bei einem pH unterhalb 2 reduziert und den Endstoff bei einem pH von unterhalb 2 abtrennt.

**Claim**

A process for the preparation of aminoanthraquinones by reduction, wherein a nitroanthraquinone is reduced with a titanium(III) salt at a pH of less than 2, and the end product is separated off at a pH of less than 2.

**Revendication**

Procédé de préparation d'amino-anthraquinones par réduction, caractérisé en ce que l'on réduit des nitroanthraquinones par des sels du titane trivalent à un pH inférieur à 2 et on sépare le produit final à un pH inférieur à 2.